# EUROPEAN PATENT APPLICATION

(11) **EP 4 442 822 A1**
(43) Date of publication of application: **09.10.2024**
(21) Application number: 23461551.6
(22) Date of filing: 05.04.2023
(51) Int. Cl.: C12N 11/14, C12N 11/02, C12P 7/06, C12N 11/12, C12N 11/04

(54) **METHOD OF OBTAINING BIOCARBON BED WITH HIGH ABSORPTION CAPACITY OF SACCHAROMYCES CEREVISIAE YEAST AND USE OF BIOCARBON BED WITH IMMOBILIZED SACCHAROMYCES CEREVISIAE YEAST**

(71) Applicant: Uniwersytet Zielonogórski, 65-417 Zielona Góra (PL)
(72) Inventor: Kulus, Katarzyna, 67-400 Wschowa (PL); Kliks, Jaroslaw, 66-120 Kargowa (PL); Korycka-Korwek, Justyna, 65-128 Zielona Gora (PL); Mrowczynska, Maria, 65-273 Zielona Gora (PL)
(74) Representative: JD&P Patent Attorneys Joanna Dargiewicz & Partners

(57) **Abstract**

The subject matter of the present invention is a method of immobilizing *Saccharomyces cerevisiae* yeast on a biocarbon bed, characterized in that it includes steps in which a biocarbon bed is produced; *Saccharomyces cerevisiae* yeast cells are brought to a logarithmic growth phase; biocarbon is mixed with *Saccharomyces cerevisiae* yeast cells.

The subject matter of the invention to also the use of a biocarbon bed with immobilized *Saccharomyces cerevisiae* yeast cells obtained by this method as a fermentation bed, preferably in the ethanol production process.

## Description

The present invention relates to a method of obtaining a biocarbon bed with high absorption capacity of *Saccharomyces cerevisiae* yeast and a use of the biocarbon bed with immobilized *Saccharomyces cerevisiae* yeast.

Biocarbon is a substance obtained by a process of pyrolysis of plants or other organic matter. It is characterized by high porosity and sorption capacity. In industrial applications, it is used to absorb odors, volatile substances, heavy metals and to decolorize liquid products.

The object of the present invention was to develop a method of obtaining a biocarbon bed with high absorption capacity of *Saccharomyces cerevisiae* yeast and immobilization of *Saccharomyces cerevisiae* yeast on the biocarbon bed.

### Prior art

The Czech patent CZ305666B6 discloses a method for producing a preparation based on biocarbon for promoting plant growth, which is based on the use of biocarbon in combination with cultured microorganisms. The phytomass intended for the preparation of biocarbon is heated in an induction heating reactor and the obtained biocarbon is then enriched with the addition of microorganisms and/or mycorrhizal fungi grown on biomass and/or fertilizers and/or hydrocolloids. The preparation obtained is used especially in agricultural and forestry practice to support the growth of higher plants.

Mexican patent application MX2012010137A describes a method for treating a biomass material, in particular a biomass material of plant origin, to pyrolyse it, comprising subjecting the biomass material to radiofrequency electromagnetic radiation, e.g. microwave radiation, while the material is mixed, in appropriate conditions to obtain the desired degree of pyrolysis. Pyrolysis temperature ranges from about 70°•C to 175°C (torrefaction). Coal, a solid product obtained in the process, used as feedstock for a coal-fired power plant. The application also relates to a compressed char product which is obtained by further treating the solid char product by mixing with a binder such as a binder, e.g. starch, and compacting the mixture to form a compressed product.

Taiwanese patent application TW202215979A discloses a method for producing biocarbon, which includes burning plant biomass materials and carbonizing plant biomass materials; grinding charred materials from plant biomass and obtaining biocarbon with a particle size in the range of 10 micrometers to 1000 micrometers. The application also relates to a feed additive containing biocarbon and microorganisms which are selected from Bacillus, photosynthetic bacteria, lactobacilli, yeast and Vibrio. Also disclosed is a method of feeding the feed, the use of a feed additive to produce an aquaculture feed, and a method for purifying water.

In turn, Chinese patent application CN113234714A relates to a microbial inoculum that promotes plant growth, including functional flora that promotes plant growth as the main ingredient, corn straw biocarbon as an adsorbent, polyvinyl alcohol as a carrier, sodium alginate and Ca(NO₃)₂ as a cross-linking agent for fixing.

Another Chinese patent application CN111670750A relates to the microbial inoculant of the Rhizophagus intraradices-biocarbon complex and the method of its production and use. The complex microbial inoculant comprises Rhizophagus intraradices and biocarbon in a mass ratio (4-15:1), where Rhizophagus intraradices is obtained by isolation from the rhizospheres of Solanaceae plants and biocarbon is rice straw biocarbon. The microbial inoculant increases the emergence rate of pepper seedlings, plant height, stem thickness, aboveground biomass, root biomass and fresh fruit weight per plant.

The next Chinese patent application CN106365139A discloses biocarbon prepared from a harvest of plants refined in a single-family backyard. The production process includes steps in which the Fe2+-enriched plant biomass is dried and ground; broken plant particles are placed in a carbon dioxide atmosphere, the temperature is raised to 800-900°C from room temperature in 150-200 minutes and held for 100-200 minutes, after which the temperature is lowered to room temperature in 150-200 minutes, and biomass is carbonized; biocarbon formed after carbonization is subjected to microwave radiation with a microwave radiation power of 300 W-700 W, and then soaked, washed and activated with zinc chloride; the biocarbon obtained after activation is dried and sodium borohydride solution is added dropwise in an ice-water bath to reduce the ferrous ions to zero valence iron; followed by washing with water until neutral pH and drying to obtain activated biocarbon.

Another Chinese patent application CN113980695A relates to a method for treating plant biomass enriched with heavy metals. Plants growing in soil contaminated with heavy metals are subjected to pyrolysis, biomass is subjected to pyrolysis to biocarbon at a temperature of 400-800°C, followed by extraction of biocarbon using a solution of nitric acid with a pH of 1-3.

Another Chinese patent application CN114456811A relates to a carbon-based soil remediation agent and a method for its production. Farmland carbon based soil remediation agent contains 10%-25% modified biocarbon and 75%-90% beneficial bacteria by weight, the beneficial bacteria include *Lactobacillus plantarum, Lactobacillus acidophilus, Saccharomyces cerevisiae, Bacillus subtilis, Bacillus licheniformis and Bacillus megatherium.*

### Summary of the invention

Thus, the subject matter of the present invention is a method of immobilizing *Saccharomyces cerevisiae* yeast on a biocarbon bed, characterized in that it includes steps in which:
a) A biocarbon bed is produced;
b) *Saccharomyces cerevisiae* yeast cells are brought to a logarithmic growth phase;
c) the biocarbon is mixed with *Saccharomyces cerevisiae* yeast cells, wherein
   in step a)
      - pomace, preferably oligosaccharide-rich deoiled flax or coconut pomace, is placed in a microwave dryer on a conveyor belt, with a belt speed of 0.15-0.25 m/minute, microwave frequency in the range of 2.40 to 2.45 GHz, for a period of 1 hour, at a power of 20 kW;
      - the obtained matter is subjected to a vacuum process in a chamber at 150°C, at a pressure of 10-400 Pa for 10 hours with the outlet valve open, followed by a thermal process according to the temperature program: 1 h - 300°C, 8 h - 650-700°C with the outlet valve closed;
      - after this time, the chamber is cooled to 200°C and the pressure is equalized to ambient pressure;
      - the obtained biocarbon is purified to remove mineral deposits;
   in step b)
      - freeze-dried *Saccharomyces cerevisiae* cells in the amount of 10 g are placed in the fermentation tank in 1 L of medium solution containing 40 g/L of glucose, 50 mg/L of magnesium ions Mg²⁺, 170 mg/L of sodium ions Na⁺, 100 mg/L of calcium ions Ca²⁺, and 2 mg/L of chromium ions Cr²⁺;
      - the mixture of cells in the medium is heated to 35°C with continuous stirring at 150 RPM and the fermentation process is carried out for 8 hours;
      - the medium is successively added to the fermentation tank in the amount of 300 mL per hour for further 3 hours;
   in step c)
      - 200 g of the biocarbon obtained in step a) is added to the fermentation tank from step b) and the fermentation is continued, adding 500 mL of medium every hour for further 3 hours;
      - another 200 g of biocarbon is added to the fermentation tank, the whole is mixed and left for 1 hour, after which the biocarbon containing the absorbed biomass is centrifuged.

Preferably, the method additionally includes step d) of encapsulating the biocarbon containing the absorbed biomass.

Preferably, in step d):
- 400 g of biocarbon containing the absorbed biomass is added to 2 L of a solution composed of: 40 g/L of glucose, 20 g/L of sodium alginate, 5 g/L of sodium carbonate, 0.1 g/L of zinc sulfate (VI) and the whole is mixed for 30 minutes at 150 RPM;
- then the biomass-containing biocarbon suspension is dispensed by microdroplets with a droplet size of 20 to 45 µL into a high-speed mixer containing a 2 wt% calcium chloride (CaCl₂) solution;
- the obtained encapsulated particles are centrifuged, washed with distilled water and separated.

Preferably, the purification of the biocarbon bed obtained in step a) includes steps in which:
- 1 kg of the biocarbon bed is placed in a tank containing 10 L of 10 wt% HCl solution;
- the mixture is stirred and heated to 40°C for 1 hour;
- the biocarbon precipitate is centrifuged and washed with 10 L of distilled water for 10 minutes;
- the precipitate is centrifuged and washing with distilled water is repeated three times;
- the obtained biocarbon precipitate is convection-dried for 10 hours at 105°C.

The subject matter of the invention is also the use of the biocarbon bed with immobilized *Saccharomyces cerevisiae* yeast cells obtained by the above-defined method as a fermentation bed, preferably in the ethanol production process.

### Brief description of the drawings

The present invention is shown in an embodiment in the drawings, where:
Fig. 1 shows a microscopic image of yeast cells immobilized on the biocarbon surface; and
Fig. 2 shows a fermentation system including: medium, feeding pump, bioreactor with the biocarbon bed containing immobilized microorganisms, and product tank.

### Detailed description of the invention

Biocarbon is characterized by varying porosity and unpredictable binding capacity of absorbed substances. In order to be able to control and standardize the sorption capacity of biocarbon and to be able to use it as a fermentation bed with known parameters, a method was developed to fix the absorbed biomass inside the pores of the biocarbon. The substance thus produced can have use both in the alcohol industry and in the food industry as a carrier for biologically active microorganisms.

### Example 1

### Biocarbon bed production

In order to obtain a biocarbon bed, the pomace, in this embodiment an oligosaccharide-rich deoiled flax or coconut pomace, was placed in a microwave dryer on a conveyor belt. The belt speed was 0.25 m/minute, the microwave frequency in the range of 2.40 to 2.45 GHz, the process duration was 1 hour, and the power was 20 kW. As a result of the above process, largely carbonized matter was obtained, which was subjected to a vacuum process in a steel chamber, at 150°C, pressure 10 Pa, for 10 hours. This process has a positive effect on the removal of volatile organic compounds. The outlet valve was then closed and the thermal process was carried out according to the temperature program: 1 h - 300°C, 8h - 700°C. After this time, the chamber was cooled to 200°C and the pressure control valve was opened to equalize the pressure with the environment. The obtained biocarbon was washed to remove mineral deposits according to the following procedure:
1 kg of biocarbon was placed in a tank containing 10 L of 10 wt% HCl solution. The mixture was stirred using a high-speed mixer and heated to 40°C. After one hour, the biocarbon precipitate was centrifuged and washed with 10 L of distilled water for 10 minutes. The precipitate was then centrifuged and washing with water was repeated three times. The biocarbon precipitate thus obtained was convection-dried for 10 hours at 105°C.

### Example 2

### Immobilization of biomass on biocarbon bed

The method of immobilizing biomass on the produced biocarbon bed is to be applied to obtain a product that could be used as a microbial carrier. In the case of the *Saccharomyces cerevisiae* yeast, the biocarbon bed so produced can serve as a fermentation bed in a continuous fermentation process.

Preferably the microorganism is brought to the so-called logarithmic growth phase before the actual immobilization process. This is a state in which there is a dynamic increase in the number of the microorganism cells. For the *Saccharomyces cerevisiae* yeast, the logarithmic (dynamic) growth phase was achieved as follows:
Freeze-dried *Saccharomyces cerevisiae* cells in the amount of 10 g were transferred to 1 dm³ of medium solution containing 40 g/L of glucose, 50 mg/L of magnesium ions Mg²⁺, 170 mg/L of sodium ions Na⁺, 100 mg/L of calcium ions Ca²⁺, and 2 mg/L of chromium ions Cr²⁺. The solution was heated to 35°C with constant stirring (150 RPM). The fermentation was carried out for 8 hours. After this time, media were successively added to the fermentation tank in the amount of 300 mL of medium per hour. In this way, the process was carried out using the fed-batch method for further 3 hours. After this time, 200 g of biocarbon was added to the total volume of 1.9 L of medium obtained, and fermentation was continued by adding 500 mL of medium every hour for further 3 hours. After 3 hours, 200 g of biocarbon was added, the total was mixed and left for 1 hour, and then the biocarbon was centrifuged.

The obtained 400 g of biocarbon containing the absorbed biomass was transferred to a beaker containing 2 L of solution composed of: 40 g/L of glucose, 20 g/L of sodium alginate, 5 g/L of sodium carbonate, 0.1 g/L of zinc sulfate (VI). The solution was stirred for 30 minutes at 150 RPM.

The biomass-containing biocarbon suspension was then dispensed by microdroplets (droplet size 20-45 µL) into a high-speed mixer containing a 2 wt% calcium chloride (CaCl₂) solution.

Interactions between the alginate and calcium ions result in the formation of coats of the sparingly soluble form of calcium alginate. Inside the coats there is a solution containing particles of biocarbon with biomass absorbed on its surface. The encapsulated particles thus produced were centrifuged and washed three times in distilled water, and then separated on sieves.

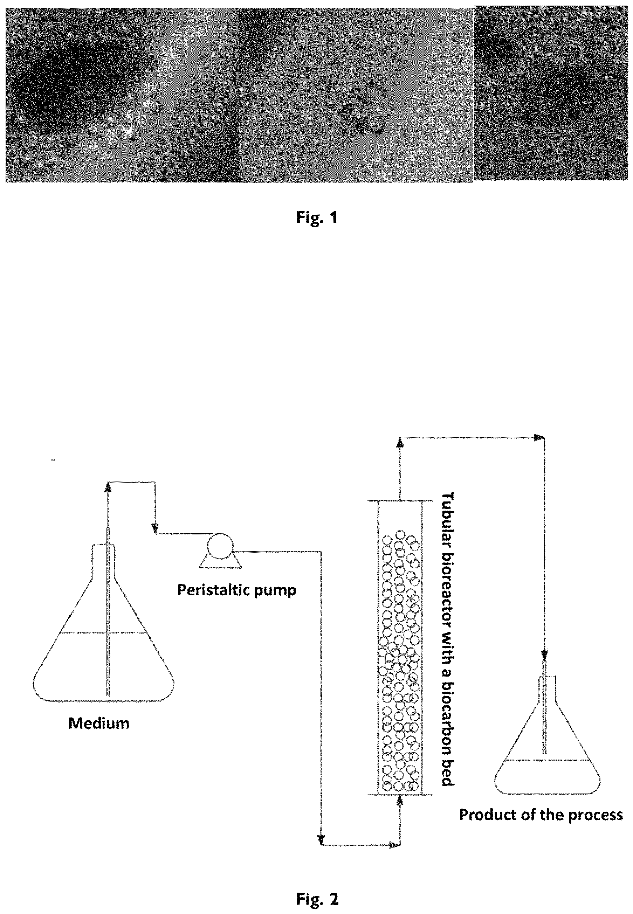

Fig. 1 shows a microscopic image of yeast cells immobilized on the biocarbon surface. As a result of interactions between calcium alginate and the porous structure of the biocarbon particles, there is effective binding of yeast cells on the surface.

The produced substrate can be subjected to convection-drying in the subsequent steps or, as a wet mass, can form the packing of a continuous tubular bioreactor.

### Example 3

### Continuous fermentation using a bed of immobilized yeast

The bioreactor system is shown in Fig. 2. The fermentation medium (substrate), the composition of which is described below, was fed via a peristaltic pump into the bioreactor containing the biocarbon bed. The fluid flow was set at 59, 71, and 83 cm³/h. The working volume of the bioreactor was 1 dm³. The retention time of the medium in the bioreactor was 17 h, 14 h, and 12 h, respectively.

### The composition of the fermentation substrate:

Distilled water 1 dm³, glucose 130 g/dm³, magnesium sulfate (VI) 200 mg/dm³, zinc chloride 30 mg/dm³, sodium hydrogen phosphate (V) 110 mg/dm³.

A continuous fermentation process was carried out with medium feed rate corresponding to the retention times of 12, 14 and 17 h. The results of the process are shown in Table 1. The results obtained show that for the same retention times of the medium in the bioreactor, a significantly higher ethanol concentration (21.6-26.1 g/dm³) was obtained in the product. Higher process dynamics were also noted. The most dynamic process occurred under conditions of intensive medium feeding (retention time 12 h) where results of 1.7928 g/dm³/h were achieved. A significantly higher concentration of biomass (4.11-4.65 g/dm³) was also recorded in the received fermentation vehicle than in the vehicle without immobilization process.

**Table 1. The end results of the fermentation process carried out on a biocarbon bed with yeast immobilization and the reference test, in which an activated carbon bed was used without yeast immobilization process.**

| Retention time of the medium in the bioreactor [h] | **Process using biocarbon and immobilized biomass according to Example 1** | | | **Process using carbon bed without immobilized yeast** | | |
|---|---|---|---|---|---|---|
| | Ethanol concentration [g/dm³] | Ethanol production rate [g/dm³/h] | Biomass concentration [g/dm³] | Ethanol concentration [g/dm³] | Ethanol production rate [g/dm³/h] | Biomass concentration [g/dm³] |
| 17 | 26.1 | 1.5399 | 4.52 | 13.5 | 0.7965 | 1.63 |
| 14 | 23.1 | 1.6401 | 4.65 | 12.7 | 0.9017 | 1.24 |
| 12 | 21.6 | 1.7928 | 4.11 | 11.3 | 0.9379 | 1.22 |

## Claims

1. A method of immobilizing *Saccharomyces cerevisiae* yeast on a biocarbon bed, **characterized in that** it includes steps in which:
a) a biocarbon bed is produced;
b) *Saccharomyces cerevisiae* yeast cells are brought to the logarithmic growth phase;
c) the biocarbon is mixed with *Saccharomyces cerevisiae* yeast cells, wherein
in step a)
- pomace, preferably oligosaccharide-rich deoiled flax or coconut pomace, is placed in a microwave dryer on a conveyor belt, with a belt speed of 0.15-0.25 m/minute, microwave frequency in the range of 2.40 to 2.45 GHz, for a period of 1 hour, at a power of 20 kW;
- the obtained matter is subjected to a vacuum process in a chamber at 150°C, at a pressure of 10-400 Pa for 10 hours with the outlet valve open, followed by a thermal process according to the temperature program: 1 h - 300°C, 8 h - 650-700°C with the outlet valve closed;
- after this time, the chamber is cooled to 200°C and the pressure is equalized to ambient pressure;
- the obtained biocarbon is purified to remove mineral deposits;
in step b)
- freeze-dried *Saccharomyces cerevisiae* cells in the amount of 10 g are placed in the fermentation tank in 1 L of medium solution containing 40 g/L of glucose, 50 mg/L of magnesium ions Mg²⁺, 170 mg/L of sodium ions Na⁺, 100 mg/L of calcium ions Ca²⁺, and 2 mg/L of chromium ions Cr²⁺;
- the mixture of cells in the medium is heated to 35°C with continuous stirring at 150 RPM and the fermentation process is carried out for 8 hours;
- medium is successively added to the fermentation tank in the amount of 300 mL per hour for further 3 hours;
in step c)
- 200 g of the biocarbon obtained in step a) is added to the fermentation tank from step b) and the fermentation is continued, adding 500 mL of medium every hour for further 3 hours;
- another 200 g of biocarbon is added to the fermentation tank, the whole is mixed and left for 1 hour, after which the biocarbon containing the absorbed biomass is centrifuged.

2. A method according to claim 1, **characterized in that** it additionally includes step d) of encapsulating the biocarbon containing the absorbed biomass.

3. The method according to claim 2, **characterized in that** in step d):
- 400 g of biocarbon containing the absorbed biomass is added to 2 L of a solution composed of: 40 g/L of glucose, 20 g/L of sodium alginate, 5 g/L of sodium carbonate, 0.1 g/L of zinc sulfate (VI) and the whole is mixed for 30 minutes at 150 RPM;
- then the biomass-containing biocarbon suspension is dispensed by microdroplets with a droplet size of 20 to 45 µL into a high-speed mixer containing a 2 wt% calcium chloride (CaCl₂) solution;
- the obtained encapsulated particles are centrifuged, washed with distilled water and separated.

4. The method according to any of the claims 1 to 3, **characterized in that** the purification of the biocarbon bed obtained in step a) includes steps in which:
- 1 kg of the biocarbon bed is placed in a tank containing 10 L of 10 wt% HCl solution;
- the mixture is stirred and heated to 40°C for 1 hour;
- the biocarbon precipitate is centrifuged and washed with 10 L of distilled water for 10 minutes;
- the precipitate is centrifuged and washing with distilled water is repeated three times;
- the obtained biocarbon precipitate is convection-dried for 10 hours at 105°C.

5. A use of the biocarbon bed with immobilized *Saccharomyces cerevisiae* yeast cells obtained by the method defined in any of the claims 1 to 4 as a fermentation bed.

6. The use according to claim 5 in the ethanol producing process.
